# EUROPEAN PATENT APPLICATION

(11) **EP 3 505 185 A1**
(43) Date of publication of application: **03.07.2019**
(21) Application number: 17857803.5
(22) Date of filing: 05.09.2017
(51) Int. Cl.: A61K 45/06, A61K 33/14, A61K 31/506, A61K 9/48, A61K 9/20, A61K 9/19, A61K 9/16, A61K 9/08, A61P 25/00

(54) **APPLICATION OF GSK3 INHIBITOR IN PREPARING A DRUG TO TREAT NIEMANN-PICK DISEASE TYPE C**

(30) Priority: 08.10.2016 CN 201610883764
(71) Applicant: Wuxi Hanqiang Pharmaceutical Technology Co., Ltd., Wuxi, Jiangsu 214000 (CN)
(72) Inventor: TANG, Ling, Nanjing Jiangsu 210042 (CN); YE, Zhijia, Nanjing Jiangsu 210042 (CN); YE, Hansen, Nanjing Jiangsu 210042 (CN); XU, Zhangzhan, Nanjing Jiangsu 210042 (CN); LIU, Xiaohao, Nanjing Jiangsu 210042 (CN)
(74) Representative: Laufhütte, Dieter
(86) International application number: PCT/CN2017/100469
(87) International publication number: WO 2018/064922

(57) **Abstract**

An application of a GSK3 inhibitor in preparation of a drug for treating Niemann-Pick disease type C. The GSK3 inhibitor can significantly improve the self-renewal and differentiation capacities of NPC1 KD or NPC1 KO hematopoietic and neural stem cells.

## Description

### Technical Field

The present invention relates to the field of medicines, and relates to applications of a GSK3 inhibitor in preparation of a drug for treating Niemann-Pick Disease Type C (NPD-C, NP-C).

### Background

NP-C is an autosomal recessive genetic disease. The incidence rate in Europe is estimated to be 1.12:100,000. The incidence rate in China is still unclear. With the improvement of diagnostic techniques, newly discovered cases increase, and the actual incidence rate is increasing. The disease occurs from infants to adults and is more common in childhood. Due to different ages of onset, the initial neurological symptoms are inconsistent, and the clinical manifestations are different. In general, the earlier the age of onset is, the heavier the pathogenic condition is. Severe onset in the neonatal period can cause death in a short term, and adult onset can be manifested as chronic neurodegenerative diseases. Typical cases are generally first manifested as systemic organ involvement, such as cholestatic jaundice in the neonatal period, hepatosplenomegaly or simple splenomegaly in the infancy or childhood, then affecting the nervous system, where symptoms of involvement in cerebellum, brainstem, basal ganglia, cerebral cortex and the like are manifested, the nervous system manifestations mainly include: rapid eye movement abnormalities, ataxia, dysmetria, dysarthria, dysphagia, and progressive dementia. Gelastic seizures, epileptic seizures, hearing impairment, and dystonia are also common. The most characteristic manifestation is Vertical Supranuclear Gaze Palsy (VGSP). The gelastic seizures are caused by sudden loss of muscle tension, and thus a standing posture cannot be maintained without conscious disturbance, and it is also one of the NP-C specific symptoms.

Under normal circumstances, NPC1 and NPC2 synergistically maintain the flow of free cholesterol in cells, so as to maintain the balance of cholesterol metabolism. Low-Density Lipoprotein (LDL) enters cells by means of pinocytosis, and is hydrolyzed by lysosomal acid lipase in late endosome and lysosome to release free cholesterol, and then NPC2 and the NPC1 proteins synergistically transport the free cholesterol to lipid membranes of organelles. NPC1 or NPC2 gene mutation leads to transport dysfunction of free cholesterol in cells, which is the initiation of NP-C pathogenesis. NPC1 and NPC2 gene analysis reveals that the pathogenic mutation is the gold standard for diagnosis of NP-C diseases. Gene testing can also be used for prenatal diagnosis. With the application of gene testing techniques, the reported NP-C cases are increasing. 95% of the NP-C cases are caused by NPC1 mutations and 5% by NPC2 mutations.

Nowadays there is no effective treatment to NP-C. The main treatment is the symptomatic treatment, and comprehensive treatment is used to improve the neurological function and quality of life of patients. At present, Miglustat is the only specific drug approved for treatment of NP-C in countries such as Europe, Australia and Japan. Long-term follow-up observations and cohort studies confirm that Miglustat can stabilize and improve the neurological symptoms of NP-C patients to prolong the life expectancy. To avoid the neurological damage from continuing to increase, the NP-C patients should be treated with Miglustat as early as possible after diagnosis. The main side effects of the drug are gastrointestinal discomfort, diarrhea and weight loss, which can be gradually alleviated after a period of administration. However, the price of Miglustat is high and most patients cannot afford it. Therefore, with the existing technology, there is still a lack of a really effective drug for treating the NP-C.

### Summary

With respect to the bottleneck of the existing technology in the drugs for treating the NP-C, the present invention states that GSK3 inhibitors (LiCl and CHIR99021) significantly improve the self-renewal and differentiation capacities of NPC1 KD or NPC1 KO hematopoietic and neural stem cells. Taking treatment of NPC1 KD hematopoietic stem cells with LiCl as an example, 10 mM LiCl can increase the proliferation of hematopoietic stem cells by 50% or more and the colony formation capacity by 30% or more. Provided is an application of a GSK3 inhibitor in preparation of a drug for treating NP-C.

The present invention adopts an NPC1 KO mouse model. From the first week, NPC1 KO mice are intraperitoneally injected with LiCl (85 mg/kg) or CHIR99021 (20 mg/kg), or the same dose is intragastrically administrated orally, and the hematopoietic function of the mice is significantly improved, and the neurodegenerative change symptoms are also significantly improved, where the effect of CHIR99021 is more obvious. More importantly, two types of GSK3 inhibitors prolong the lifespan of NPC1 KD mice by 3-6 weeks. The effectiveness of the GSK3 inhibitor composition is clarified, and thus the basis for parenteral administration and oral administration is provided.

The present invention provides GSK3 inhibitors for preparing a drug for treating NP-C diseases, including lithium salt, CHIR99021, 6-BIO, TDZD-8, and SB-216763.

The pharmaceutical composition of the present invention includes an oral preparation or a non-oral preparation. The oral preparation includes tablets, capsules, granules, syrups, and solutions. The non-oral preparation includes injections, lyophilized preparations, and the like.

The clinical dose of the GSK3 inhibitor of the present invention is adjustable according to factors such as the conditions and age of the patient.

The carrier excipients used in the pharmaceutical composition of the present invention are different excipients commonly used in the preparation.

### Brief Description of the Drawings

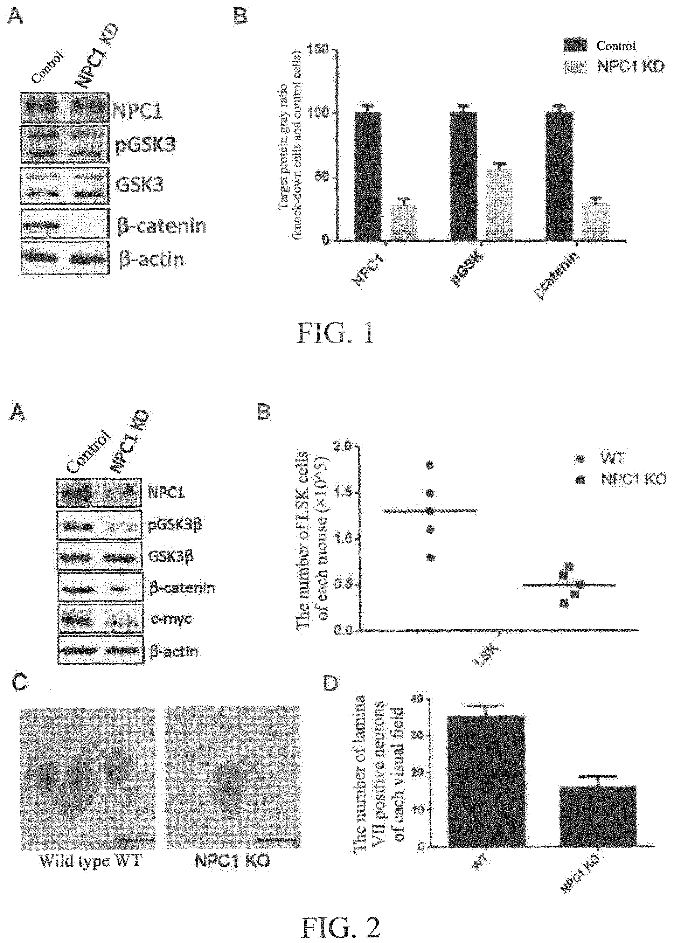

FIG. 1 shows that GSK3 phosphorylation of knock-down cells of the NPC1 gene is reduced, and β-catenin expression is down-regulated. NPC1 shRNA transfects 293T cells, the NPC1 expression is down-regulated by about 75%, the GSK3 phosphorylation is down-regulated by about 43%, and the β-catenin expression is down-regulated by 72%. FIG. A shows a western blotting onetime result, and FIG. B shows a statistical result of three western blotting result gray values.
FIG. 2 shows that hematopoietic stem cells of NPC1 KO mouse cell are reduced, and neurons are reduced. The NPC1 expression of hematopoietic stem cells (LSK, lineage-Sca-1+c-kit+) of the NPC1 KO mice is completely inhibited, pGSK3 phosphorylation is reduced, wnt signaling pathway effector molecule β-catenin expression is reduced, wnt signaling pathway target gene c-myc expression is reduced (A), mouse bone marrow LSK cells are significantly reduced (B), and mouse neurons (lamina VII positive cells) are significantly reduced (C and D).

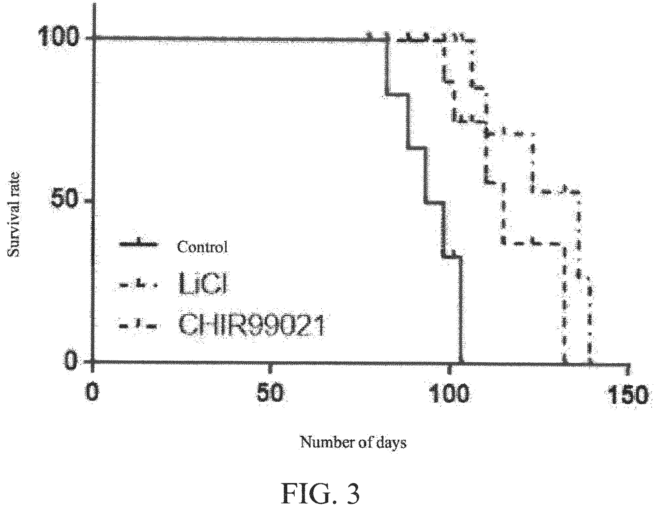

FIG. 3 shows that the GSK3 inhibitor prolongs the lifespan of NPC1 KO mice. NPC1 KO mice are divided into three groups (a control group, a LiCl treatment group and a CHIR99021 treatment group), with five mice in each group. From the first week after birth, the mice in the LiCl treatment group and the CHIR99021 treatment group are intraperitoneally injected with LiCl (85 mg/kg) and CHIR99021 (20 mg/kg) per day respectively, the mice in the control group are intraperitoneally injected with normal saline, and the survival rate of the mice is continuously observed.

### Detailed Description

### Embodiment 1. Preparation of NPC cell models and discovery of disease mechanisms

### 1. Method

Cell preparation:
1) Fibroblasts from an NP-C patient: skin fibroblasts of the NP-C patient are taken; and
2) NPC1 gene expression knock-down (KD) human and mouse cells: lentivirus expressing human and mouse NPC1 shRNA is prepared, lentivirus infects human embryonic kidney 293T and Mouse Embryonic Fibroblast (MEF), positive infected cells are screened by puromycin, and qPCR and western blotting validate the effect of NPC1 gene expression knock-down, to obtain the NPC1 KD cell model.

The testing indexes are NPC1, GSK3, and β-catenin.

### 2. Results

1) Compared with normal subjects, the NPC1 expression in fibroblasts of the NP-C patient is significantly reduced;
2) compared with normal cells, the expression of NPC1 in NPC1 gene knockdown cells is down-regulated by more than 70%; and
3) fibroblasts and NPC1 KD cells of the NP-C patient: the GSK-3 β activity is increased, the β-catenin stability is decreased, and the expression is reduced.

The results show that the β-catenin expression in NPC1 KD or NPC1 KO cells is decreased, the wnt signaling pathway transduction is disordered, and the cell growth and differentiation are abnormal. It is indicated that NPC1 KD or NPC1 KO cells are reliable models for screening drugs, and drugs acting on the wnt signaling pathway are used for treating NPC.

### Embodiment II. Preparation of NPC mouse model and verification of pathogenesis

### 1. Method

An NPC1 gene knock-out (KO) mouse preparation method: the NPC1 genes are knocked out by CRISPY_Cas9 to obtain NPC1-/- mice.

The measurement indexes are functions and phenotypes of hematopoietic and neurological stem cells, as well as symptoms and signs.

### 2. Results

1) The hematopoietic and neurological phenotypes of the NPC1 KO mouse model are similar to those of the NP-C patient, with hematopoietic dysfunction and neurodegenerative changes. The lifespan of mice is no more than 14 weeks in the absence of effective interventions, which is similar to the clinical patients; and
2) the β-catenin expressions of neural stem cells, hematopoietic stem cells and liver parenchyma cells of the NPC1 KO mice are significantly reduced, and the expression of target genes in the wnt signaling pathway is down-regulated.

The results show that: by using the animal model, it is verified that the wnt signaling pathway transduction disorder is an important cause of corresponding dysorganoplasia and phylogenetic disorder in the NPC1 KO mice, and the manifestations thereof are similar to the clinical manifestations, the drug screening reliability is further indicated, and it is further indicated that the drug for the wnt signaling pathway can be used for treating the NP-C.

### Embodiment III. Effect of the GSK3 inhibitor for improving the NP-C cell model

### 1. Method

According to the method of Embodiment I, NPC model cells are prepared, and hematopoietic stem cells and neural stem cells of the NPC1 KO mice are isolated.

Experimental procedures and measurement indexes are: treatment of NPC1 KD and NPC1 KO cells with GSK3 specific inhibitors, i.e., LiCl and CHIR99021, and observation of changes in cell proliferation and stem cell self-renewal and differentiation.

### 2. Results

LiCl (the concentration of 10MM) and CHIR99021 (the concentration of 3uM) significantly improve the self-renewal and differentiation capacities of NPC1 KD or NPC1 KO hematopoietic and neural stem cells. Taking treatment of NPC1 KD hematopoietic stem cells with LiCl as an example, the proliferation capacity of hematopoietic stem cells is increased by 50% or more, and the colony formation capacity is increased by 30% or more.

The results show that the GSK3 inhibitor significantly improves the self-renewal and differentiation capacities of the NP-C cells, and can be used for treating the NPC.

### Embodiment IV. Effect of the GSK3 inhibitor for improving the NPC mouse model

### 1. Method

The NPC mouse model is prepared according to the method of Embodiment II, and LiCl (the dose of 85 mg/kg) or CHIR99021 (the dose of 20 mg/kg) is intraperitoneally injected every day from the first week.

The testing indexes are phenotypes of hematological and neurological systems, as well as symptoms and signs.

### 2. Results

1) The hematopoietic function of the mice is significantly improved, and the neurodegenerative change symptoms are also significantly improved, where the effect of CHIR99021 is more obvious; and
2) the two GSK3 inhibitors prolong the lifespan of the NPC1 KD mice for 3-6 weeks.

The results show that the GSK3 inhibitors improve the symptoms of the NPC1 KO mice and prolong the lifespan thereof, which indicates that the GSK3 inhibitors have a significant therapeutic effect on the NPC.

## Claims

1. An application of a GSK3 inhibitor in preparation of a drug for treating Niemann-Pick disease type C.

2. An application of a composition formed by the GSK3 inhibitor according to claim 1, serving as an active ingredient, and a pharmaceutically acceptable excipient in preparation of a drug for treating Niemann-Pick disease type C.

3. The GSK3 inhibitor according to claims 1 and 2, wherein the GSK3 inhibitor is lithium salt, CHIR99021, 6-BIO, TDZD-8, and SB-216763.

4. The composition according to claims 1 and 2, wherein the composition comprises an oral preparation or a non-oral preparation; the oral preparation comprises tablets, capsules, granules, syrups, and solutions; and the non-oral preparation comprises injections, lyophilized preparations, and the like.

5. The application according to claims 1 and 2, wherein a clinical dose of the GSK3 inhibitor is adjustable according to factors such as conditions and age of a patient.
